# EUROPEAN PATENT SPECIFICATION

(11) **EP 1 205 152 B1**
(45) Date of publication and mention of the grant of the patent: **29.09.2004**
(21) Application number: 00500231.6
(22) Date of filing: 10.11.2000
(51) Int. Cl.: A61B 17/70

(54) **Spinal column deformity correction device**
Gerät zur Korrektur einer Deformation der Wirbelsäule
Dispositif de correction d'une déformation de la colonne vertébrale

(43) Date of publication of application: 15.05.2002
(73) Proprietor: Lafitt, S.A., 46988 Paterna (Valencia) (ES)
(72) Inventor: Perez Millan, Luis, C/. Villa de Bilbao, 40, 46988 Paterna (Valencia) (ES); Atienza Vicenta, Carlos M., C/. Villa de Bilbao 40, 46988 Paterna (Valencia) (ES); Forriol Pico, Joaquin, C/. Villa de Bilbao, 40, 46988 Paterna (Valencia) (ES); Molla Domenech, Fernando, C/. Villa de Bilbao, 40, 46988 Paterna (Valencia) (ES); Mas Gomez, Francesc J., C/. Villa de Bilbao, 40, 46988 Paterna (Valencia) (ES)
(74) Representative: Flaccus, Rolf-Dieter, Dr.

(56) References cited:
- DE-A- 2 845 647
- DE-A- 19 738 968
- US-A- 4 269 178

## Description

The purpose to which the invention refers consists of a Spinal column malformation correction device.

The malformations for the correction of which both the procedure and the device are applied consist of curvature of the longitudinal axis of the spine when this should be anatomically straight, with both (procedure and device) being apt for correcting any malformation, pathological or traumatic, whatever its origin.

On the other hand, the claimed device admits slight variants that do not alter or impair its essentiality, thus constituting "a group of inventions interrelated in such a way that they integrate a unique general inventive concept".

### HISTORY

Different attachment techniques of the attaching elements posterior to the vertebras are known, which make up the spinal column malformation correction systems by means of lateral displacement of the vertebras.

The preferential fastening of said attaching elements is located on the vertebral laminas as these are the most resistant mooring places of the posterior area of the rachis and, furthermore, the less affected by the osteoporosis processes that weaken the bone structure.

The failure of various types of instrumentation usually occurs either due to pull-out of the attaching elements or due to the excessive magnitude of the loads applied to the rachis.

The known and most used attaching elements are the following:
Transpedicular screws: The placing of the screws through the vertebral peduncles implies a high risk of neurological damage as the peduncle can break, damaging the nearby nervous roots or the spinal cord itself. On the other hand, the strength required to remove them is relatively little.
Sublaminar wires: These do not permit the transmission of axial loads to the tying bars, thus causing sliding along them when compression, lateral inclination and flexion displacements are made. Due to the reduced contact surface between the wire and the bone they tend to cut the vertebral laminas.
Claws: These give rise to a connection that is unable to resist flector and torsion moments as they allow turning movements, although they provide resistance to posterior removal and longitudinal traction-compression forces.

US-A-4 269 178 discloses an apparatus useful for the treatment of scoliosis. This apparatus comprises two metallic rods which are placed on either side of the curvature of the spinal column. One of the rods acts as a distraction system, the other rod acts as a compression system. Each rod is provided with hooks which penetrate into the inter-articular space and act as anchoring devices. In the peak region of the scoliotic curvature, both rods may be connected by a transverse tensioning device to achieve a better correction of the curvature.

DE-A-2 854 647 refers to a device for correcting scoliotic malformations. The device comprises a rotatable rod which is attached to the spinal column on the concave side of the curvature. The rod is provided with a wire which is fixed to a vertebra located in the peak region of the curvature. Upon rotation, the wire is wound around the rod, thus straightening the curvature. The wire is fixed to a vertebra by using a screw. The rotatable rod is affixed to the spinal column by means of wire brackets.

### DESCRIPTION OF THE INVENTION

The purpose of the invention constituting the object of this Patent consists in overcoming the inconvenience characteristic of the known procedures and devices for spinal column malformation correction by means of lateral moving of the vertebras described above. It has been conceived and designed specifically for this priority purpose.

The procedure is of the type used for application to longitudinal metallic bars that serve as a support when nearing to them the elements attached to the vertebras, thus obtaining their lateral displacement. It includes the following operations:
Fastening of a longitudinal metallic bar on the concave side of the spinal column curvature by means of pincers attached to the laminar or pedicular zone of the vertebras and linked to lateral bar-pincer connectors located on top and beneath the deformation, near the ends of the bar.
Union by means of independent metallic wires of each intermediate pincer with a wire-bar connector linked to the bar, retaining an end of each wire at the corresponding pincer by means of prisoner screws and passing the opposite end through the connector.
Progressive traction of all the wires from their free ends that have passed through the connectors, by means of tighteners anchored on the wire-bar connectors, until obtaining approximation of the intermediate pincers and the vertebras to which they are attached to the longitudinal bar, thus completing correction of the curvature.
Mounting of a second longitudinal bar on the convexity side of the curvature to bilaterally stabilize the spinal column, attaching this second bar to the vertebras by means of other pincers.
Replacement of the wires by lateral pincer-bar connectors when this is possible due to the proximity of the pincer to the bar.

The device for putting the described procedure into practice consists of the following elements:
Pincers attached to the vertebras through their laminar or pedicular zone, formed by two curved claws with opposite concavities, one of which forms part of the body of the pincer and the other independent, with a cylindrical lateral prolongation which on passing tightly through the body of the pincer allows it to make axial or turning displacements to adapt the relative position of the two claws to the shape and sizes of the zone of the vertebra where it is fastened. There is a prisoner screw for immobilization of the movable claw in the suitable position, as well as another prisoner screw to retain insertion of the connecting prolongation of the pincer-bar connectors and tautening wire through-orifice for lateral displacement of the vertebras.
Pincer-bar connectors that have an orifice or groove for tight housing of the bar with a prisoner screw for its immobilization in the suitable position and a lateral cylindrical prolongation for connection to the corresponding'pincer.
Longitudinal metallic bar held on its ends by pincer-bar connectors.
Wire-bar connectors formed by a polyhedral body with beveled edges, crossed by a groove or longitudinal orifice through which the bar passes and to which it is attached by means of a prisoner screw, and by an orifice with two paths (side inlet and upper outlet), joined by curved transition through which the wire passes and a prisoner screw to retain it. This layout facilitates surgical access to the free end of the wire so that it can be tautened.

Braided metallic wires thanks to the progressive pulling of which by means of conventional tighteners the approximation stress of the vertebras to the bar is caused. For this purpose a low cylindrical butt is provided on the end projecting through the body of the pincer.

### BRIEF DESCRIPTION OF THE DRAWINGS

To complete the description of the invention and facilitate the interpretation of its formal, structural and functional characteristics, attached are drawings in which the different aspects of a preferred performance of the device for putting into practice the spinal column malformation correction procedure constituting the object of this Patent.

In said drawings:

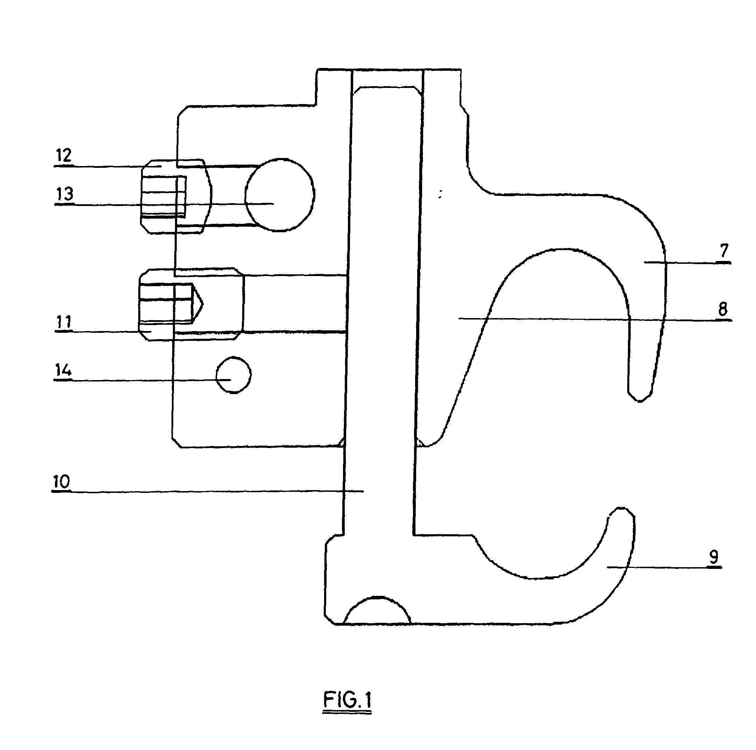

Figure 1 presents a longitudinal section of a pincer fastened to the vertebras.

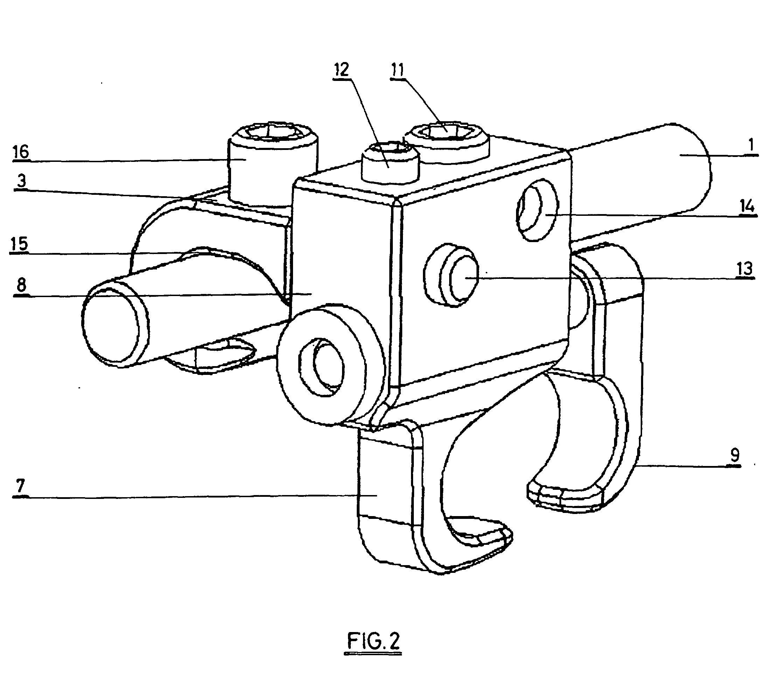

Figure 2 represents a perspective view of the union between the bar and pincer by means of a connector.

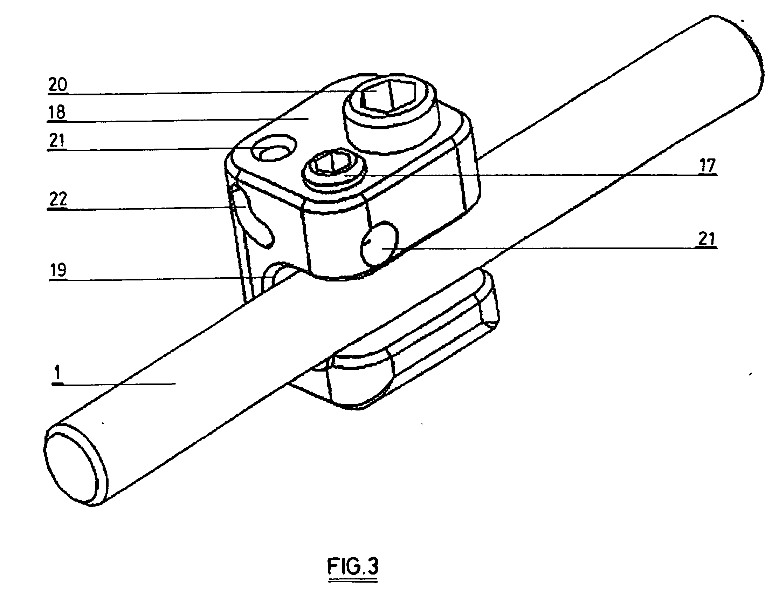

Figure 3 represents a perspective view of the union of a wire-bar connector to the bar;

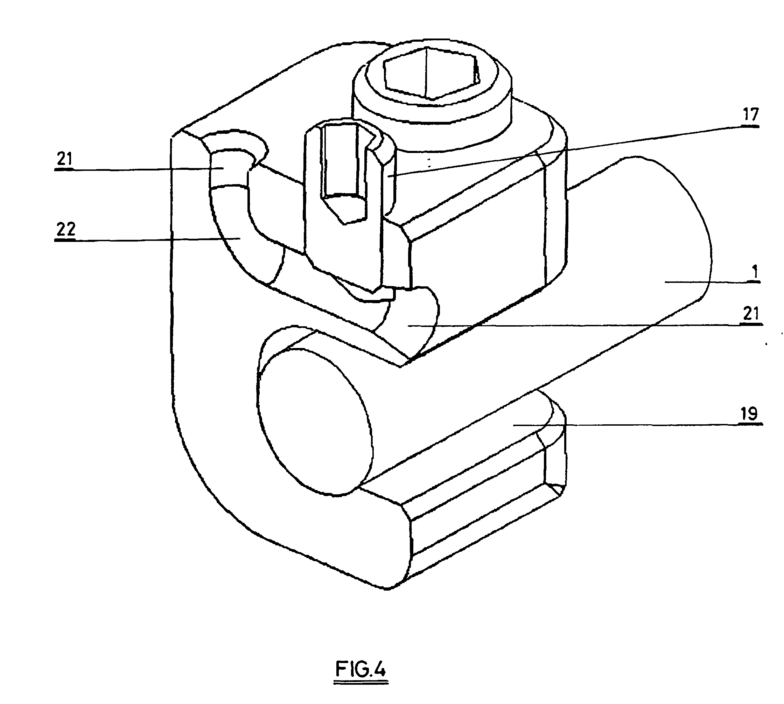

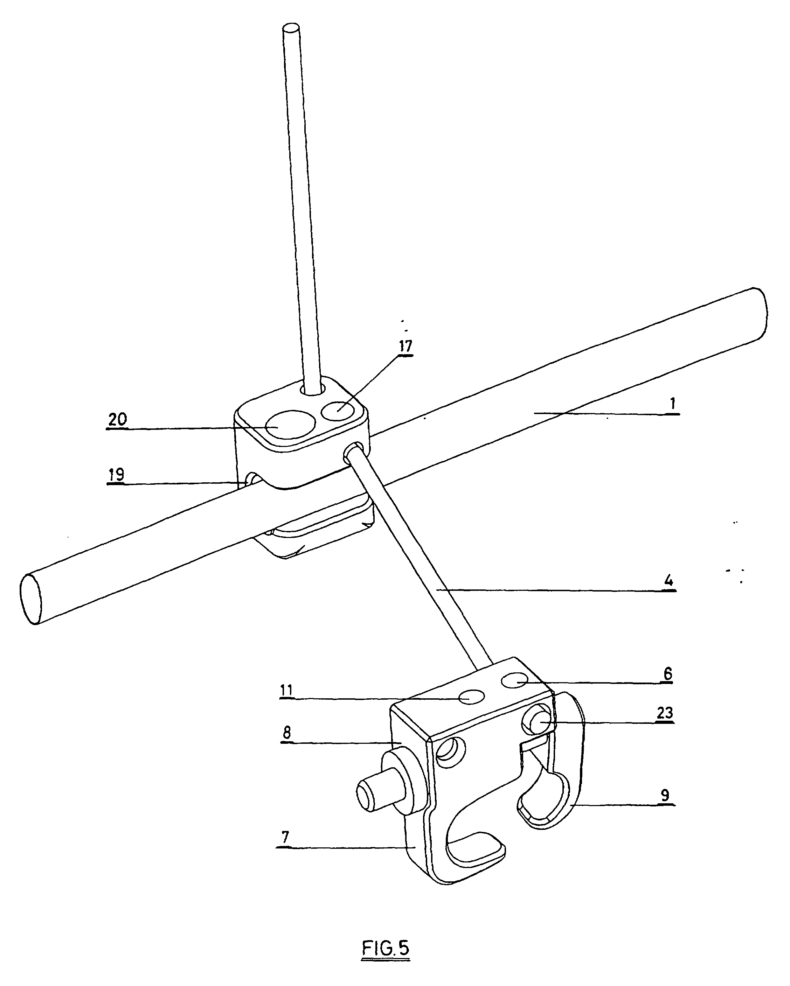

figure 4 a transversal section of the same through a vertical plane that diametrically cuts the wire through-orifice; and figure 5 the active position of a wire-bar connector mechanically joining the bar and pincer by means of a wire.

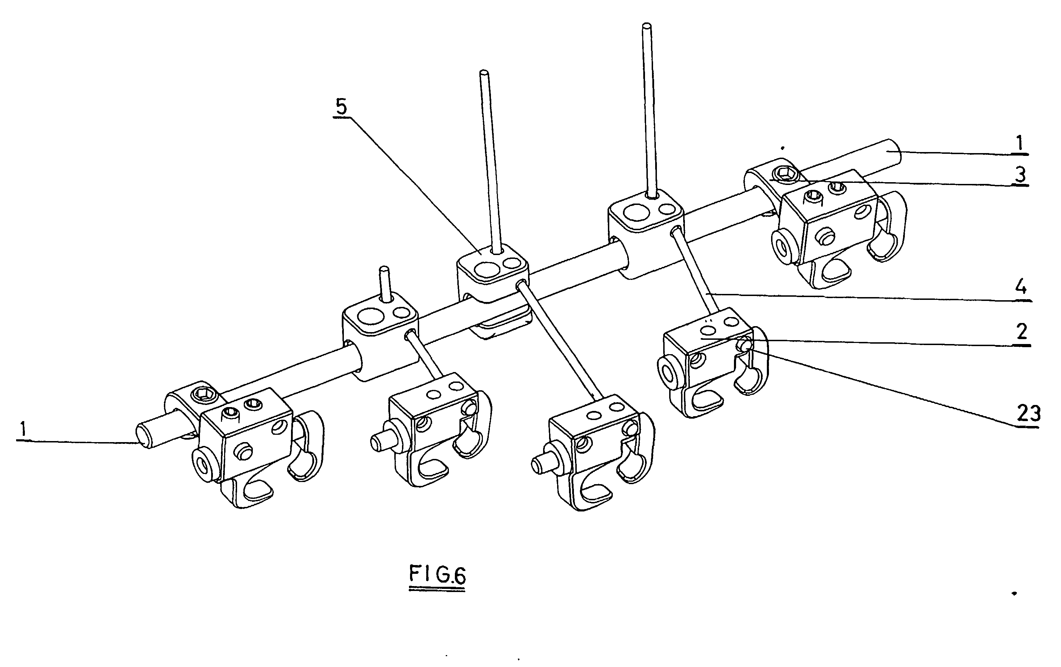

Figure 6 is a general perspective of the correcting device, showing the relative positions of the end pincers linked to the bar by means of pincer-bar connectors and of the intermediate pincers mechanically joined to the bar by means of respective wires through wire-bar cables.

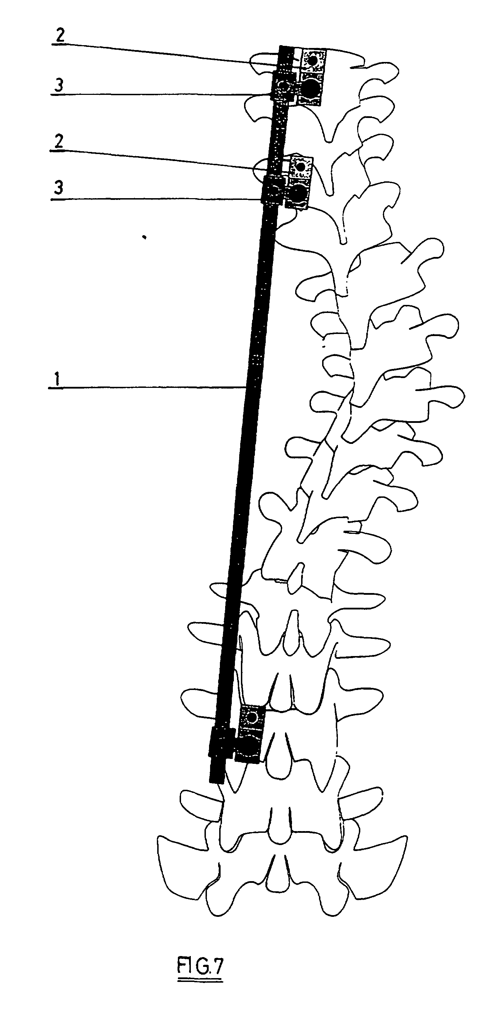

Figure 7 schematically represents the connection of the longitudinal to the column by means of end pincers and pincer-bar connectors.

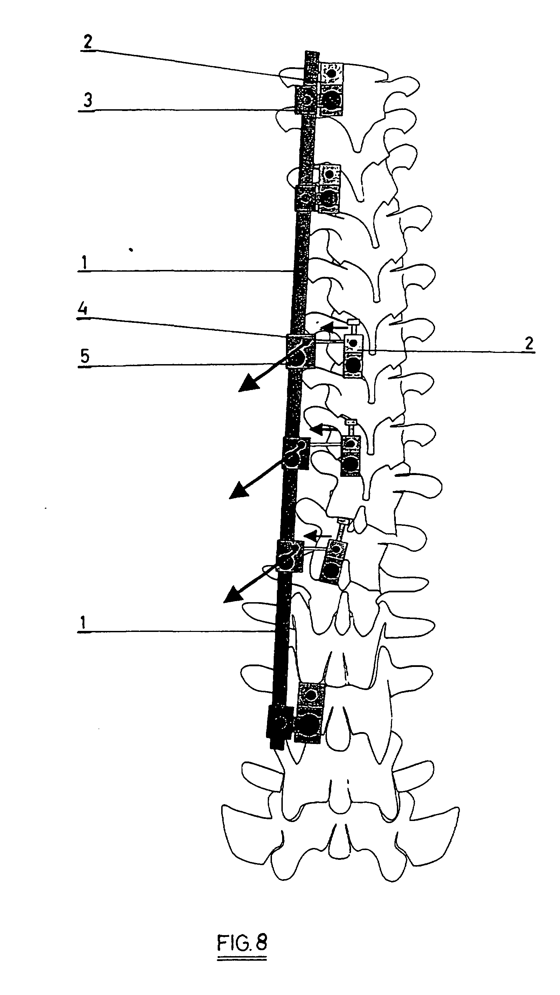

Figure 8 shows the application of the procedure by means of the device, with correction of the curvature already obtained through lateral displacement of the vertebras and their approximation to the bar, thanks to the pulling of the wires on the intermediate pincers.

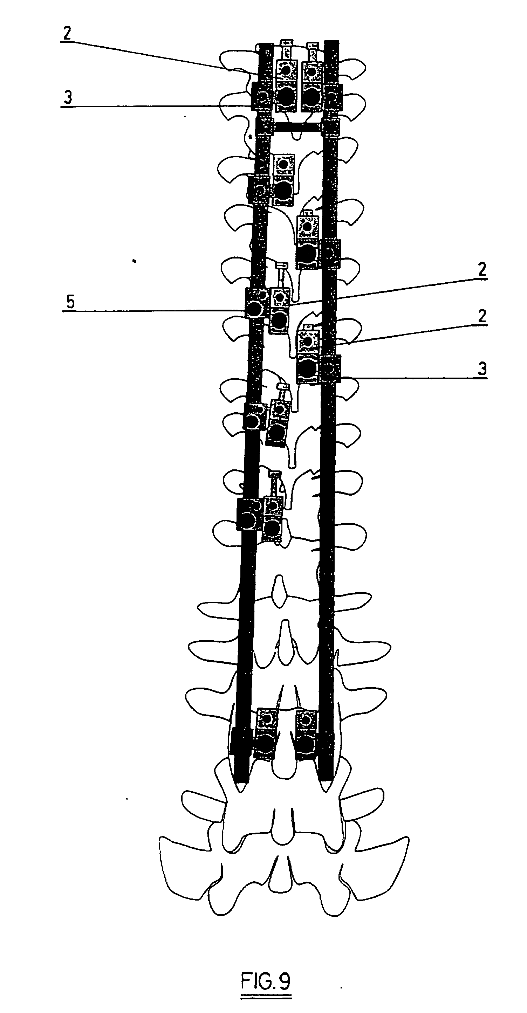

Figure 9 shows bilateral stabilization of the column with a second longitudinal bar.

### DESCRIPTION OF A PREFERRED PERFORMANCE

In order to clearly show the nature and scope of the advantageous application of the Spinal column malformation correction device, constituting the object of the invention as described in claim 1, the following is a description of its application and structure, making reference to the drawings which, on representing a preferred performance of the device and its components for information purposes, must be considered in the widest sense and not limitative of the application and content of the claimed invention.

The correction is of the type that uses, for its application, longitudinal metallic bars serving as a support for nearing the elements attached to the vertebras to them.

It includes the following operations:
Fastening of a longitudinal metallic bar (1) on the concave side of the spinal column curvature by means of pincers (2) attached to the laminar or pedicular zone of the vertebras and linked to lateral bar-pincer connectors (3) located on top and beneath the deformation, near the ends of the bar (1).
Union by means of independent metallic wires (4) of each intermediate pincer (2) with a wire-bar connector (5) linked to the bar (1), retaining an end of each wire at the corresponding pincer by means of prisoner screws (6) and passing the opposite end through the connector (5).
Progressive traction of all the wires (4) from their free ends that have passed through the connectors (5), by means of tighteners anchored on the wire-bar connectors, until obtaining approximation of the intermediate pincers (2) and the vertebras to which they are attached to the longitudinal bar, thus completing correction of the curvature. means of other pincers.
Replacement of the wires (4) by lateral pincer-bar connectors (3) when this is possible due to the proximity of the pincer to the bar.

The device for putting the described procedure into practice consists of the following elements:
Pincers (2) attached to the vertebras, formed by two curved claws with opposite concavities, one of which (7) forms part of the body (8) of the pincer and the other (9) independent, with a cylindrical lateral prolongation (10) which on passing tightly through the body (8) of the pincer allows it to make axial or turning displacements to adapt the relative position of the two claws to the shape and sizes of the zone of the vertebra where it is fastened. There is a prisoner screw (11) for immobilization of the movable claw (9) in the suitable position, as well as another prisoner screw (12) to retain insertion of the connecting prolongation (13) of the pincer-bar connectors (3) and tautening wire (4) through-orifice (14) for lateral displacement of the vertebras.
Lateral pincer-bar connectors (3) that have an orifice or groove (15) for tight housing of the bar with a prisoner screw (16) for its immobilization in the suitable position and a lateral cylindrical prolongation (13) for connection to the corresponding pincer (2).
Wire-bar connectors (5) formed by a polyhedral body (18) with beveled edges, crossed by a groove or longitudinal orifice (19) through which the bar (1) passes and to which it is attached by means of a prisoner screw (20), and by an orifice (21) with two paths (side inlet and upper outlet), joined by curved transition (22) through which the wire (4) passes and a prisoner screw (17) to retain it.

Braided metallic wires (4) the progressive pulling of which nears the pincers (2) and the vertebras to which these are attached to the bar (1). For this purpose a low cylindrical butt (23) is provided on the end projecting through the body (8) of the pincer (2).

## Claims

1. Device for use in a procedure for the correction of spinal column malformation, comprising one or two longitudinal metallic bars and a plurality of anchoring means which are detachably connected to each of said bars and which can be attached to the vertebras, **characterized in that** it comprises
- pincers (2) serving as said anchoring means, wherein each of said pincers is formed by a pair of curved claws with opposite concavities, and wherein one claw (9) of said pair of claws is moveable and can be tightened against the other claw (7) which is fixed;
- pincer-bar connectors (3) suitable for connecting a first group of said pincers to said bar(s), wherein said pincer-bar connectors and said first group of pincers serve to fasten said bar(s) to the spinal column;
- wire-bar connectors (5) serving to connect a second group of pincers to said bar(s) by means of metallic wires (4) fixed to said pincers and passing through said connectors, thus allowing the application of traction forces on said wires and pincers for correcting the curvature of the spinal column.

2. Device in accordance with claim 1, **characterized in that** said pincers are attachable to the laminar or pedicular zone of the vertebras.

3. Device in accordance with claim 1 or 2, **characterized in that** said moveable claw (9) comprises a cylindrical lateral prolongation (10) passing tightly through the body (8) of the pincer, thus allowing this claw to make axial or turning movements to adapt the relative positions of the two claws to the shape and sizes of the vertebras.

4. Device in accordance with claim 3, **characterized in that** said pincer comprises a locking screw (11) for immobilizing the movable claw in a suitable position.

5. Device in accordance with any one of the preceding claims, **characterized in that** the body of said pincers is provided with a bore (14) through which wire (4) passes, and the end of wire (4) which projects through said bore of the body is provided with a cylindrical butt (23), and said wire is a braided metallic wire.

6. Device in accordance with any one of claims 1 to 4, **characterized in that** the body of said pincers is provided with a bore (14) through which wire (4) passes, and a locking screw (6) for retaining the end of the wire in a fixed position.

7. Device in accordance with any one of the preceding claims, **characterized in that** a bore is provided in the body of said pincer for inserting a connecting prolongation (13) which extends from one of said pincer-bar connectors, and a second locking screw (12) is provided in the body of the pincer for immobilizing the pincer in a suitable position on said prolongation.

8. Device in accordance with any one of claims 1-6, **characterized in that** said pincer-bar connectors (3) are provided with an orifice or groove (15) suitable for receiving the bar (1), and with a locking screw (16) for immobilizing the pincer-bar connector in a suitable position on said bar (1).

9. Device in accordance with claim 8, **characterized in that** said pincer-bar connectors are provided with a connecting prolongation (13) which passes through a bore provided in the body (8) of a pincer attached to said connecting elongation, and a second locking screw (12) is provided in the body of the pincer for immobilizing the pincer in a suitable position on said connecting elongation.

10. Device in accordance with any one of the preceding claims, **characterized in that** said wire-bar connector comprises
- a longitudinal orifice or groove (19) formed in the body (18) of said connector, said orifice or grove being suitable for receiving the bar (1);
- a third locking screw (20) by which bar (1) passing through said orifice or groove can be fixed in a suitable position;
- a tube-like, curved channel (22) which is formed inside the body (18) of said connector and which serves to receive wire (4) passing through the inlet and outlet (21) openings of said channel;
- a locking screw (17) for retaining wire (4) in a fixed position.

11. Device in accordance with any one of the preceding claims, **characterized in that** said wire-bar connector comprises a polyhedral body (18) with bevelled edges.

12. Device in accordance with any one of the preceding claims, **characterized in that** each pincer-bar connector is connected to a pincer (2);
- a plurality of wire-bar connectors (5) attached to the intermediate portion of said bar which lies between said end portions, wherein each wire-bar connector is connected to a pincer by means of a wire or cable (4).

13. Device in accordance with claim 12, **characterized in that** it is affixed to a spinal column which exhibits a curvature, such that
- said metallic bar (1) is fastened on the concave side of the curvature of the spinal column, the upper end portion of the bar being located on top of the curvature and the lower end portion of the bar being located underneath the curvature;
- at least one pincer-bar connector is attached, in use, to the upper end portion of said bar (1), each pincer-bar connector being connected to a pincer (2);
- at least one pincer-bar connector is attached, in use, to the lower end portion of said bar (1), each pincer-bar connector being connected to a pincer (2);
- a plurality of wire-bar connectors (5) is attached to the intermediate portion of said bar which lies between said end portions, wherein each wire-bar connector is connected to an intermediate pincer by means of a wire or cable (4) which passes through the connector;
- said pincers being able to be anchored to vertebras of the spinal column.

14. Device in accordance with any one of the preceding claims, **characterized in that** it comprises a pair of longitudinal bars (1), each having a plurality of pincer-bar connectors and a plurality of wire-bar connectors attached thereto, wherein each of said connectors is connected to a pincer.

15. Device in accordance with claim 14, **characterized in that** both bars (1) are joined together mechanically.

## Patentansprüche

1. Gerät zur Verwendung in einem Verfahren zur Korrektur von Wirbelsäulenfehlbildungen, umfassend eine oder zwei longitudinale Metallstangen und eine Vielzahl von Verankerungsmitteln, die lösbar mit jeder einzelnen der Stangen verbunden sind und an den Wirbeln befestigt werden können, dadurch gekenazeichnet, dass sie umfasst
- Zangen (2), die als die besagten Verankerungsmittel dienen, wobei jede der Zangen aus einem Paar gekrümmter Krallen mit entgegengesetzter Konkavität gebildet und eine Kralle (9) des Krallenpaars beweglich ist und an die andere, feste Kralle (7) herangezogen werden kann;
- Zange-und-Stange-Verbinder (3), die für die Verbindung einer ersten Gruppe der besagten Zangen mit der Stange bzw. den Stangen geeignet sind, wobei diese Zange-und-Stange-Verbinder und die besagte erste Gruppe von Zangen der Befestigung der Stange bzw. Stangen an der Wirbelsäule dienen;
- Draht-und-Stange-Verbinder (5), die dazu dienen, eine zweite Gruppe von Zangen mit der/den Stange(n) mittels Metalldrähten (4) zu verbinden, welche an den Zangen befestigt sind und durch die Verbinder hindurch verlaufen, wodurch das Anlegen von Zugkräften an die besagten Drähte und Zangen zur Korrektur der Wirbelsäulenkrümmung ermöglicht wird.

2. Gerät nach Anspruch 1, **dadurch gekennzeichnet, dass** die Zangen an den Plättchen- oder Pedikel-Zonen der Wirbel befestigt werden können.

3. Gerät nach Anspruch 1 oder 2, **dadurch gekennzeichnet, dass** die besagte bewegliche Kralle (9) einen seitlichen zylinderförmigen Fortsatz (10) umfasst, der in engem Sitz durch den Körper (8) der Zange hindurch verläuft, wodurch axiale oder Drehbewegungen der Kralle ermöglicht werden, um die relativen Positionen der beiden Krallen an die Form und die Größenabmessungen der Wirbel anzupassen.

4. Gerät nach Anspruch 3, **dadurch gekennzeichnet, dass** die besagte Zange eine Befestigungsschraube (11) zum Feststellen der beweglichen Kralle in einer geeigneten Position umfasst.

5. Gerät nach einem der vorhergehenden Ansprüche, **dadurch gekennzeichnet, dass** der Körper der besagten Zangen mit einer Bohrung (14) versehen ist, durch welche der Draht (4) verläuft, und dass das Ende des Drahtes (4), welches aus der Bohrung des Körpers herausragt, mit einem zylindrischen Endstück (23) versehen ist, und dass der besagte Draht ein umflochtener Metalldraht ist.

6. Gerät nach einem der Ansprüche 1 bis 4, **dadurch gekennzeichnet, dass** der Körper der Zangen mit einer Bohrung (14) versehen ist, durch welche der Draht (4) hindurch verläuft, sowie mit einer Befestigungsschraube (6) zum Festklemmen des Drahtendes in einer festen Position.

7. Gerät nach einem der vorhergehenden Ansprüche, **dadurch gekennzeichnet, dass** in dem Körper der Zange eine Bohrung vorgesehen ist, die zum Einführen eines Verbindungsfortsatzes (13) dient, der von einem der Zange-und-Stange-Verbinder ausgeht, und dass eine zweite Befestigungsschraube (12) in dem Körper der Zange vorgesehen ist, um die Zange in einer geeigneten Position auf dem Fortsatz festzustellen.

8. Gerät nach einem der Ansprüche 1-6, **dadurch gekennzeichnet, dass** die Zange-und-Stange-Verbinder (3) mit einer für die Aufnahme der Stange (1) geeigneten Öffnung oder Nut (15) versehen sind, sowie mit einer Befestigungsschraube (16) zum Festsetzen des Zange-und-Stange-Verbinders in einer geeigneten Position auf der besagten Stange (1).

9. Gerät nach Anspruch 8, **dadurch gekennzeichnet, dass** die Zange-und-Stange-Verbinder mit einem Verbindungsfortsatz (13) versehen sind, der durch eine Bohrung hindurch verläuft, die in dem Körper (8) einer an diesem Verbindungsfortsatz befestigten Zange vorgesehen ist, und dass eine zweite Befestigungsschraube (12) in dem Körper der Zange vorgesehen ist, um die Zange in einer geeigneten Position auf dem Verbindungsfortsatz festzusetzen.

10. Gerät nach einem der vorhergehenden Ansprüche, **dadurch gekennzeichnet, dass** der Draht-und-Stange-Verbinder umfasst:
- eine longitudinale Öffnung oder Nut (19), die in dem Körper (18) des besagten Verbinders ausgebildet ist, wobei diese Öffnung oder Nut zur Aufnahme der Stange (1) geeignet ist;
- eine dritte Befestigungsschraube (20), durch welche die Stange (1), die durch die Öffnung oder Nut hindurch verläuft, in einer geeigneten Position befestigt werden kann;
- ein rohrförmiger, gekrümmter Kanal (22), der im Inneren des Körpers (18) des Verbinders ausgebildet ist und der zur Aufnahme des Drahtes (4) dient, welcher durch die Eingangs- und die Ausgangsöffnung (21) des Kanals hindurch verläuft;
- eine Befestigungsschraube (17) zum Festklemmen des Drahtes (4) in einer festen Position.

11. Gerät nach einem der vorhergehenden Ansprüche, **dadurch gekennzeichnet, dass** der Draht-und-Stange-Verbinder einen polyedrischen Körper (18) mit gebrochenen Kanten umfasst.

12. Gerät nach einem der vorhergehenden Ansprüche, **dadurch gekennzeichnet, dass** jeder Zange-und-Stange-Verbinder mit einer Zange (2) verbunden ist;
eine Mehrzahl von Draht-und-Stange-Verbindern (5) an dem intermediären Abschnitt der Stange, der sich zwischen den Endbereichen befindet, befestigt ist, wobei jeder Draht-und-Stange-Verbinder mittels eines Drahtes oder eines Kabels (4) mit einer Zange verbunden ist.

13. Gerät nach Anspruch 12, dadurch gekenazeichnet, dass es derart an einer eine Krümmung aufweisenden Wirbelsäure befestigt ist, dass
- die Metallstange (1) auf der konkaven Seite der Krümmung der Wirbelsäule befestigt ist, wobei der obere Endbereich der Stange oberhalb der Krümmung und der untere Endbereich der Stange unterhalb der Krümmung angeordnet ist;
- wenigstens ein Zange-und-Stange-Verbinder im Gebrauch an dem oberen Endbereich der Stange (1) befestigt ist, wobei jeder Zange-und-Stange-Verbinder mit einer Zange (2) verbunden ist;
- wenigstens ein Zange-und-Stange-Verbinder im Gebrauch an den unteren Endbereich der Stange (1) befestigt ist, wobei jeder Zange-und-Stange-Verbinder mit einer Zange (2) verbunden ist;
- eine Mehrzahl von Draht-und-Stange-Verbindern (5) an dem intermediären Bereich der Stange, der sich zwischen den besagten Endbereichen befindet, befestigt ist, wobei jeder Draht-Stange-Verbinder mit einer intermediären Zange verbunden ist, und zwar mittels eines Drahtes oder Kabels (4), der/das durch den Verbinder hindurch verläuft;
- wobei die Zangen an den Wirbeln der Wirbelsäule verankerbar sind.

14. Gerät nach einem der vorhergehenden Ansprüche, **dadurch gekennzeichnet, dass** es ein Paar longitudinaler Stangen (1) umfasst, an denen jeweils eine Mehrzahl von Zange-und-Stange-Verbindern und eine Mehrzahl von Draht-und-Stange-Verbindern befestigt ist, wobei jeder der besagten Verbinder mit einer Zange verbunden ist.

15. Gerät nach Anspruch 14, **dadurch gekennzeichnet, dass** beide Stangen (1) mechanisch miteinander verbunden sind.

## Revendications

1. Dispositif destiné à être utilisé dans une opération de correction d'une déformation de la colonne vertébrale, qui comprend une ou deux barres métalliques longitudinales et une pluralité de moyens d'ancrage qui sont reliés de manière détachable à chacune desdites barres et qui peuvent être attachés aux vertèbres, **caractérisé en ce qu'**il comprend:
- des pinces (2) utilisées comme dits moyens d'ancrage, chacune desdites pinces étant formée par deux mâchoires incurvées dont les concavités sont opposées, une mâchoire (9) desdites deux mâchoires pouvant être déplacée et serrée contre l'autre mâchoire (7) qui est fixe,
- des raccords (3) pince-barre convenant pour relier un premier groupe desdites pinces à ladite ou auxdites barres, lesdits raccords pince-barre et ledit premier groupe de pinces servant à attacher ladite ou lesdites barres à la colonne vertébrale,
- des raccords (5) fil-barre qui servent à relier un deuxième groupe de pinces à ladite ou auxdites barres au moyen de fils métalliques (4) fixés auxdites pinces et qui traversent lesdits raccords pour ainsi permettre d'appliquer des forces de traction sur lesdits fils et sur lesdites pinces pour corriger la courbure de la colonne vertébrale.

2. Dispositif selon la revendication 1, **caractérisé en ce que** lesdites pinces peuvent être attachées à la zone laminaire ou à la zone pédiculaire des vertèbres.

3. Dispositif selon les revendications 1 ou 2, **caractérisé en ce que** ladite mâchoire mobile (9) comprend un prolongement latéral cylindrique (10) qui traverse étroitement le corps (8) de la pince en permettant ainsi à cette pince d'exécuter des déplacements axiaux ou de rotation pour adapter les positions relatives des deux pinces à la forme et à la taille des vertèbres.

4. Dispositif selon la revendication 3, **caractérisé en ce que** ladite pince comprend une vis de verrouillage (11) qui immobilise la pince mobile dans une position appropriée.

5. Dispositif selon l'une quelconque des revendications précédentes, **caractérisé en ce que** le corps desdites pinces est doté d'un alésage (14) traversé par un fil (4), et l'extrémité du fil (4) qui déborde dudit alésage du corps étant dotée d'un arrêt cylindrique (23), et ledit fil étant un fil métallique tressé.

6. Dispositif selon l'une quelconque des revendications 1 à 4, **caractérisé en ce que** le corps desdites pinces est doté d'un alésage (14) traversé par le fil (4) et d'une vis de verrouillage (6) qui retient l'extrémité du fil dans une position fixe.

7. Dispositif selon l'une quelconque des revendications précédentes, **caractérisé en ce qu'**un alésage est prévu dans le corps de ladite pince pour insérer un prolongement de raccordement (13) qui s'étend à partir de l'un desdits raccords pince-barre, une deuxième vis de verrouillage (12) étant prévue dans le corps de la pince pour immobiliser la pince dans une position appropriée sur ledit prolongement.

8. Dispositif selon l'une quelconque des revendications 1 à 6, **caractérisé en ce que** lesdits raccords pince-barre (3) sont dotés d'un orifice ou d'une rainure (15) convenant pour recevoir la barre (1), et d'une vis de verrouillage (16) qui immobilise le raccord pince-barre en position appropriée sur ladite barre (1).

9. Dispositif selon la revendication 8, **caractérisé en ce que** lesdits raccords pince-barre sont dotés d'un prolongement de raccordement (13) qui traverse un alésage ménagé dans le corps (8) d'une pince attachée audit prolongement de raccordement, une deuxième vis de verrouillage (12) étant prévue dans le corps de la pince pour immobiliser la pince en position appropriée sur ledit prolongement de raccordement.

10. Dispositif selon l'une quelconque des revendications précédentes, **caractérisé en ce que** ledit raccord fil-barre comprend:
- un orifice ou une rainure longitudinales (9) formés dans le corps (18) dudit raccord, ledit orifice ou ladite rainure convenant pour recevoir la barre (1),
- une troisième vis de verrouillage (20) qui permet de fixer en position appropriée la barre (1) qui traverse ledit orifice ou ladite rainure,
- un canal incurvé (22) de type tubulaire qui est formé à l'intérieur du corps (18) dudit raccord et qui sert à recevoir le fil (4) qui traverse les ouvertures d'entrée et de sortie (21) dudit canal et
- une vis de verrouillage (17) qui maintient le fil (4) dans une position fixe.

11. Dispositif selon l'une quelconque des revendications précédentes, **caractérisé en ce que** ledit raccord fil-barre comprend un corps polyédrique (18) à arêtes chanfreinées.

12. Dispositif selon l'une quelconque des revendications précédentes, **caractérisé en ce que**:
- chaque raccord pince-barre est relié à une pince (2),
- plusieurs raccords fil-barre (5) sont attachés à la partie intermédiaire de ladite barre qui est située entre lesdites parties d'extrémité, chaque raccord fil-barre étant relié à une pince au moyen d'un fil ou câble (4).

13. Dispositif selon la revendication 12, **caractérisé en ce qu'**il est fixé à une colonne vertébrale qui présente une courbure de manière à ce que :
- ladite barre métallique (1) est fixée sur le côté concave de la courbure de la colonne vertébrale, la partie d'extrémité supérieure de la barre étant située au sommet de la courbure et la partie d'extrémité inférieure de la barre étant située en dessous de la courbure,
- au moins un raccord pince-barre est fixé en utilisation à la partie d'extrémité supérieure de ladite barre (1), chaque raccord pince-barre étant relié à une pince (2),
- au moins un raccord pince-barre est attaché en utilisation à la partie d'extrémité inférieure de ladite barre (1), chaque raccord pince-barre étant relié à une pince (2),
- plusieurs raccords fil-barre (5) sont reliés à la partie intermédiaire de ladite barre qui est située entre lesdites parties d'extrémité, chaque raccord fil-barre étant relié à une pince intermédiaire au moyen d'un fil ou câble (4) qui traverse le raccord et
- lesdites pinces peuvent être ancrées sur les vertèbres de la colonne vertébrale.

14. Dispositif selon l'une quelconque des revendications précédentes, **caractérisé en ce qu'**il comprend deux barres longitudinales (1) qui sont chacune dotée d'une pluralité de raccords pince-barre et d'une pluralité de raccords fil-barre qui y sont attachés, chacun desdits raccords étant relié à une pince.

15. Dispositif selon la revendication 14, **caractérisé en ce que** les deux barres (1) sont reliées l'une à l'autre mécaniquement.
